Europäisches Patentamt

European Patent Office

Office européen des brevets

(19)

(11) Veröffentlichungsnummer : **0 069 310**
**B1**

(12) EUROPÄISCHE PATENTSCHRIFT

(45) Veröffentlichungstag der Patentschrift :
18.09.85

(21) Anmeldenummer : 82105694.2

(22) Anmeldetag : 26.06.82

(51) Int. Cl.⁴ : **C 07 D215/30, A 01 N 43/42**

(54) Herstellung und Verwendung eines Chinolinderivats, sowie ein solches Derivat enthaltendes Präparat.

(30) Priorität : 06.07.81 FR 8113249

(43) Veröffentlichungstag der Anmeldung :
12.01.83 Patentblatt 83/02

(45) Bekanntmachung des Hinweises auf die Patenterteilung : 18.09.85 Patentblatt 85/38

(84) Benannte Vertragsstaaten :
CH DE FR GB IT LI NL

(56) Entgegenhaltungen :
CHEMICAL ABSTRACTS, Band 81, Nr. 7, 19. August
1974, Seite 382, Nr. 37485k, Columbus Ohio (USA);
Kolloid-Zeitschrift für Polymere S. 155-161 (31/10/66)
Patent-Kokai (Laid-open) No. 43983/1974
Die Akte enthält technische Angaben, die nach dem
Eingang der Anmeldung eingereicht wurden und die
nicht in dieser Patentschrift enthalten sind.

(73) Patentinhaber : F. HOFFMANN-LA ROCHE & CO.
Aktiengesellschaft

CH-4002 Basel (CH)
CH DE GB IT LI NL
LA QUINOLEINE et ses Dérivés
43 rue de Liège
F-75008 Paris (FR)
FR

(72) Erfinder : Muller, Bernard
19 rue du Maréchal Galliéni
F-78100 St-Germain-en-Laye (FR)
Erfinder : Piat, René
204 Chemin de Clers
F-76230 Bois-Guillaume (FR)
Erfinder : Rensing, Cornelis
21 Route des Essarts
F-76350 Oissel (FR)
Erfinder : Trajin, Jean-Paul
35 bis rue des Frères Nicolle
F-76000 Rouen (FR)

(74) Vertreter : Aschert, Hubert et al
Grenzacherstrasse 124 Postfach 3255
CH-4002 Basel (CH)

EP 0 069 310 B1

## 0 069 310

**Beschreibung**

Die vorliegende Erfindung betrifft die Verwendung der β'-Form von Kupferoxinat als Antifungus-Wirkstoff und antibakterieller Wirkstoff, insbesondere für den Schutz von Saatgut, ein Verfahren zur Herstellung dieser Substanz, sowie wässrige stabilisierte Präparate, welche diese Substanz enthalten.

Es ist seit langem bekannt, dass Kupferoxinat, d. h. der Komplex von Cu (II) mit 8-Hydroxychinolin, biozide Eigenschaften aufweist. Diese Substanz wird insbesondere als Fungizid bei der Behandlung von Getreidesaatgut verwendet.

Diese Substanz weist verschiedene Vorteile auf, insbesondere ist sie nicht irritierend, bildet keine besonderen Probleme bei ihrer Handhabung, ist nur sehr schwach toxisch, praktisch nicht flüchtig und sehr stabil.

Aufgrund dieser Eigenschaften ist diese Substanz somit besonders für ihre Verwendung auf dem phytosanitären Gebiet geeignet.

Das im Handel befindliche Kupferoxinat hat insbesondere eine interessante Wirkung gegen Fusariosen, gegen Septoriose und gegen Getreidebrand ; seine Wirkung gegen Helminthosporiose ist jedoch nur sehr schwach.

Der vorliegenden Erfindung lag die Aufgabe zugrunde, eine Substanz zu finden, welche die interessanten Eigenschaften des Kupferoxinats mit einer erhöhten Wirksamkeit und einem grösseren Wirkungsspektrum in sich vereint.

Es wurde nun gefunden, dass sich diese Substanz nicht unter den Kupferoxinatderivaten sondern im Kupferoxinat selbst präsentiert. Seit den Publikationen von Palenik Acta Cryst., 1964, 17 (6), 687-95, und von Suito, Arakawa, Kobayashi, Kolloid-z.z. Polym., 1966, 212 (2), 155-161 ist es bekannt, dass Kupferoxinat eine polymorphe Verbindung ist, d. h. dass sie in verschiedenen kristallinen Formen auftritt.

Bisher hat man sieben kristalline Formen von Kupferoxinat festgestellt, nämlich die Formen α, α', β, β', β", γ und γ'. Die Formen α, α', γ und γ' können als reine Laboratoriumskuriositäten angesehen werden, während die β-Form die unter normalen Umständen und bei der üblichen Verwendung stabilste Form ist. Dementsprechend war es auch diese β-Form, in welcher das Kupferoxinat angewandt wurde und seine Wirkung entfaltete.

Man hat nun überraschenderweise gefunden, dass die β'-Form, welche als instabil gilt und eine Uebergangsform zwischen der bei normalen Bedingungen stabilen β-Form und der bei hohen Temperaturen stabilen β"-Form darstellt, ein Aktivitätsspektrum aufweist, welches viel grösser ist als das der β-Form und ausserdem als Antifungus-Wirksubstanz auch noch wirksamer ist als die β-Form.

Es war nun noch notwendig, ein Verfahren zu finden, mittels welchem die Herstellung der β'-Form von Kupferoxinat sowie die Herstellung von stabilisierten Präparaten ermöglicht wird. In Gegenwart von Feuchtigkeit und insbesondere in einem wässrigen Milieu geht nämlich die β'-Form in die β-Form über.

Das erfindungsgemässe Verfahren zur Herstellung von Kupferoxinat in β'-Form ist dadurch gekennzeichnet, dass man :

a) ein Säureadditionssalz von 8-Hydroxychinolin mit einem Cu (II)-Salz in Wasser umsetzt ;

b) eine Base zusetzt bis der pH zwischen 2 und 9, vorzugsweise zwischen 2 und 3,5 liegt ;

c) den gebildeten Niederschlag abtrennt ; und

d) den so abgetrennten Niederschlag bei einer Temperatur zwischen 40 und 220 °C dehydratisiert.

Demgegenüber wird gemäss einem bekannten Verfahren (C.A. Bd. 81, Nr. 7, Seite 382, Nr. 37485 ; japanische Patentpublikation Nr. 43983/1974) das Kupfersalz von 8-Hydroxychinolin (Kupferoxinat) dadurch erhalten, dass man ein Gemisch enthaltend ein Alkalimetallsalz von 8-Hydroxychinolin (erhalten durch Alkalischmelze von 8-Hydroxychinolin) mit Wasser verdünnt, das unlösliche Alkalimetallsalz von 8-Hydroxychinolin abfiltriert, mit Wasser erhitzt, nochmals filtriert, dem Filtrat eine wässrige Kupfersulfatlösung zusetzt, den pH durch Zugabe einer Säure auf 2-6 einstellt und das Kupfersalz von 8-Hydroxychinolin abfiltriert und wäscht. Das erhaltene feuchte Produkt enthält, wie festgestellt wurde, das Kupferoxinat in β-Form zusammen mit Verunreinigungen wie Kupfersulfat und Natriumsulfit. Das bei 60 °C getrocknete Produkt besteht im wesentlichen aus Kupferoxinat in β-Form, etwa 1 % β"-Kupferoxinat und den obigen Verunreinigungen.

Das Kupferoxinat in β'-Form ist eine Verbindung der Formel $(C_9H_6ON)_2Cu$, welche in Form eines olivgrünen Feststoffes auftritt, welcher stark elektrostatisch ist und eine fadenförmige Mikrostruktur aufweist.

Die Röntgenbeugungslinien in Dezimalgraden sind : (2θ) = 7,15 ; 11,95 ; 13 ; 14,7.

Im Gegensatz zur grossen Mehrheit der organischen Verbindungen, welche man in reiner Form mittels physikalischer Reinigungsmethoden erhält, sind die physikochemischen Charakteristika der Chelate von Oxin (8-Hydroxychinolin) und insbesondere des Chelats von Kupfer (II) vollständig bestimmt durch : die Qualität der verwendeten Ausgangsmaterialien, die Zusammensetzung der Reaktionspartner und die Reaktionsbedingungen, einschliesslich des Waschens und der Dehydratation.

Die Reaktion erfolgt in wässriger Phase, wobei man in der Stufe a) vorzugsweise eine wässrige Lösung von Kupfer (II)-Sulfat mit einer wässrigen Lösung von Oxinsulfat (Sulfat des 8-Hydroxychinolins) umsetzt, wobei man die letztere Verbindung in einem geringen stöchiometrischen Ueberschuss (beispielsweise weniger als 5 Molprozent) verwendet.

2

# 0 069 310

Anstelle des bevorzugten Oxinsulfats können auch andere Säureadditionssalze des Oxin, wie das Nitrat und das Phosphat verwendet werden und anstelle des bevorzugten Sulfats andere Cu(II)-Salze, wie das Nitrat und das Phosphat.

Die Reaktion wird vorzugsweise bei einer Temperatur unterhalb 40 °C, insbesondere unterhalb 20 °C und oberhalb 0 °C, beispielsweise bei 10 °C durchgeführt.

Die aus der Reaktion resultierende Azidität wird in situ mittels einer Base bei derselben Temperatur neutralisiert. Hierzu verwendet man vorzugsweise Ammoniak, obwohl auch andere Basen, wie beispielsweise Alkalimetallhydroxide oder Erdalkalimetallhydroxide oder organische Basen verwendet werden können.

Der pH-Wert am Ende der Reaktion ist von entscheidender Bedeutung, da man durch Einstellung dieses pH-Wertes auf 2,0 bis 3,5, insbesondere auf 2,5 bis 2,8 sich nicht nur des bei der Neutralisation des 8-Hydroxychinolinsalzes entstehenden Oxin, sondern auch des Grossteils von Oxinaten anderer Metalle entledigen kann, welche in den Reagenzien vorhanden sind (Fe, Ni, Ca, Mg).

Die Dehydrationsstufe d) wird vorzugsweise bei einer Temperatur zwischen 60 und 160 °C während einer Zeitdauer zwischen 120 Min. und 0,5 Sek., insbesondere zwischen 80 und 140 °C während einer Zeitdauer von 20 Min. bis 0,5 Sek. durchgeführt, wobei natürlich bei erhöhter Temperatur die Reaktionszeit entsprechend kürzer ist. Wenn man das Kupferoxinat in seiner β'-Form zulange einer erhöhten Temperatur aussetzt, wandelt es sich in die β''-Form um, welche sich ihrerseits wieder bei Raumtemperatur in die stabile β-Form umwandelt. So sind beispielsweise nach 275 Std. bei 30 °C in Gegenwart von Wasser 80 % der β''-Form in die β-Form umgewandelt.

Die nach den obigen Angaben erhaltene β'-Form des Kupferoxinats weist einen Gehalt von weniger als 1 % Verunreinigungen auf, was im Hinblick auf seine Anwendung auf dem phytosanitären Gebiet besonders interessant ist.

Wie bereits erwähnt, wandelt sich die β'-Form des Kupferoxinats in Gegenwart von Wasser in die β-Form um. Nun wird aber das Kupferoxinat in den meisten Anwendungsfällen in einem wässrigen Medium verwendet, insbesondere in Form von konzentrierten oder verdünnten wässrigen Brühen. Dies würde natürlich eine sehr rasche Umwandlung der β'-Form in die β-Form zur Folge haben.

Um das Kupferoxinat in β'-Form bequem anwenden zu können, ist es daher notwendig, Präparate zur Verfügung zu haben, beispielsweise in flüssige Form überführbare Pulver oder wässrige Suspensionen, welche problemlos gelagert werden können und welche mindestens 24 Stunden vor ihrer Anwendung verdünnt werden können, ohne ihre Aktivität zu verlieren.

Die vorliegende Erfindung bezieht sich daher weiters auf stabilisierte Präparate, welche als Wirkstoff, allenfalls neben anderen Wirkstoffen, die β'-Form des Kupferoxinats und einen Hydratationsinhibitor enthalten, welcher die Hydratation dieser β'-Form verhindert.

Als derartige Hydratationsinhibitoren für die β'-Form des Kupferoxinats können genannt werden Polykondensate vom Kresolphenoplasttyp und vom Formaldehydphenoplasttyp mit niedrigem Molekulargewicht, Polykondensate von Alkylnaphthalinsulfonsäuren mit Formaldehyd und deren Salze, Arylsulfonsäuren (Benzolsulfonsäure, Toluolsulfonsäure, Naphthalinsulfonsäure) und deren Salze, Polyacrylsäuren, sowie deren Homologe und deren Salze, Lignosulfonsäure und Lignosulfonate, welche oberflächenaktive, dispergierende und stabilisierende Eigenschaften aufweisen.

Diese Stabilisierungsmittel können im allgemeinen dadurch gekennzeichnet werden, dass sie einerseits eine gewisse Pufferwirkung aufweisen und dass sie andererseits funktionelle Gruppen, insbesondere OH und/oder $NH_2$ und/oder $SO_3H$, enthalten, welche in der Lage sind, schwache Bindungen mit dem Kupferatom einzugehen. Als Hydratationsinhibitoren können daher insbesondere genannt werden : das Sulfoxin (die 8-Hydroxy-chinolin-5-sulfonsäure) und dessen Salze, sowie die Halogensulfoxine, insbesondere die 7-Jod-8-hydroxy-chinolin-5-sulfonsäure und deren Salze.

Ferner weisen noch Polyvinylalkohol, Alkyl- und Hydroxyalkylcellulosen, Carboxyalkylcellulosen, Polyvinylpyrrolidon, Polyacrylamide, Polysaccharide, wie Stärken, Hemicellulosen, Gummen, Polypeptide, insbesondere Casein und Caseinate, sowie Milchpulver, eine ausgesprochene stabilisierende Wirkung auf und inhibieren auch teilweise die Hydratation der β'-Form. Weiterhin tragen diese Substanzen noch zur Erhöhung der Haftfähigkeit der mit ihnen hergestellten Brühen bei.

Die Konzentration der als Stabilisierungsmittel verwendeten Substanzen hängt von der Art dieser Substanzen ab und auch von der Art des herzustellenden Präparates. Im allgemeinen kann die Konzentration dieser stabilisierenden Substanzen zwischen 0,1 und 20 Gewichtsprozent betragen. Die folgenden Formulierungen sind Beispiele von erfindungsgemässen Präparaten :

Präparat für die Behandlung von oberirdischen Pflanzenteilen, verwendbar in verdünnter wässriger Brühe :

| | |
|---|---|
| Kupferoxinat-β' | 50 % |
| Natriumpolynaphthylmethansulfonat | 2 % |
| Natriumlignosulfonat | 3 % |
| Sulfoxin (8-Hydroxychinolin-5-sulfonsäure) | 1 % |
| Calciumcarbonat (Kreide) | 19 % |
| Kaolin | 25 % |

3

Kombinationspräparat für die Behandlung von Saatgut, verwendbar in Form von wässriger konzentrierter Brühe :

| | |
|---|---:|
| Kupferoxinat-β' | 15 % |
| Lindan | 25 % |
| Anthrachinon | 25 % |
| Natriumpolynaphthylmethansulfonat | 1 % |
| Sulfoxin | 0,2 % |
| Kaolin | 14,8 % |
| Calciumcarbonat (Kreide) | 10 % |
| Polyvinylalkohol | 5 % |
| Rouge 53 − 1 = [(4-Hydroxy-1-naphthalenyl)azo]-4'-chlor-5'-methylbenzolsulfonsäure-Bariumsalz | 4 % |

Präparat für die Behandlung von oberirdischen Pflanzenteilen verwendbar in Form einer verdünnten wässrigen Lösung :

| | |
|---|---:|
| Kupferoxinat β' | 400 g |
| Natriumpolynaphthylmethansulfonat | 15 g |
| Natriumlignosulfonat | 25 g |
| Sulfoxin | 10 g |
| Aethylenglykol | 50 g |
| Kolloidales Kieselgel | 25 g |
| Antischaumsilikon | 2 g |
| Polysaccharid | 2 g |
| Wasser, ad | 1 Liter |

Das Kupferoxinat ist eine verhältnismässig weiche und leicht zerreibbare Substanz und kann durch Schlagen oder Reiben in eine Teilchengrösse übergeführt werden, welche unterhalb der Erkennbarkeit im optischen Mikroskop liegt, d. h. im Bereich zwischen $10^{-4}$ und $10^{-5}$ cm. Für eine derartige Zerkleinerung können alle Arten von Zerkleinerungsmaschinen verwendet werden, welche eine Ultramikromisierung ermöglichen, insbesondere Strahlmikronisiervorrichtungen (Luft oder Dampf). Vorzugsweise werden zum Zwecke der Zerkleinerung Kugelmühlen mit horizontaler oder vertikaler Achse verwendet, wobei das zu zerkleinernde Material in Form einer wässrigen Suspension vorliegt, deren Konzentration an Oxinat zwischen 1 und 80 %, vorzugsweise zwischen 20 und 50 %, beträgt. Wenn man beispielsweise Kugelmühlen mit Kugeln von 1,5 mm Durchmesser verwendet, so erhält man praktisch eine kolloidale Lösung. Bei diesem Zerkleinerungsvorgang werden zweckmässig alle Hilfsstoffe zugesetzt, welche notwendig sind um die Stabilität zu erhöhen, sowie weitere Hilfsstoffe welche die Mikronisierung fördern.

Die vorliegende Erfindung betrifft auch die Anwendung des Kupferoxinats in seiner β'-Form, wobei dieses vorzugsweise weniger als 1 % an Verunreinigungen aufweist, als Antifungusmittel und als antibakterielles Mittel, insbesondere für den Schutz von Pflanzen und Saatgut. Dieses Produkt kann jedoch auch für den Schutz von Futtermitteln, von Leder, von Cellulosematerialien, wie beispielsweise Papierstoff, von Holz, von Farben, von Leimen, von Geweben und auch für den Schutz von Kohlenwasserstoffen verwendet werden.

Die erfindungsgemässen fungiziden Präparate können etwa 5-20 Gew.% β'-Kupferoxinat und etwa 0,5-5 % Hydrationsinhibitor enthalten, wobei der Rest auf 100 % Trägermaterial ist.

Die folgenden Beispiele zeigen Herstellung und Anwendung des Kupferoxinats in β'-Form.

## Beispiel 1

Herstellung der polymorphen Form β $(C_9H_6ON)$ 2 Cu · 2$H_2O$

Bei 10 °C wird einer Lösung von 142 g (0,568 8 Mol) von Kupfersulfat-Pentahydrat in 1 200 ml Wasser im Verlauf von 30 Minuten unter kräftigem Rühren eine Lösung von 232,11 g (0,568 8 Mol + 0,5 %) Oxinsulfat (8-Hydroxychinolin-sulfat) in 1 000 ml Wasser zugesetzt. Sofort nach dem Zusatz des ersten Tropfens der Oxinsulfatlösung erscheint das Kupferoxinat als zartgrüner Feststoff und es kann mittels Mikroskop der Uebergang in die polymorphen Formen α → γ → β beobachtet werden.

Die Reaktion ist nicht exotherm. Unter Beibehaltung der Ausgangstemperatur fügt man tropfenweise 85 % der theoretisch erforderlichen Menge an Ammoniak (170 ml, D 20/4 = 0,924) zu, bis sich der pH-Wert auf einen Wert zwischen 2,5 und 2,8 eingestellt hat (ca. 10 ml). Unter leichtem Rühren lässt man die Temperatur bis auf Raumtemperatur ansteigen. Hierauf wird das gebildete Produkt, welches in Form von hexagonalen Plättchen von etwa 20 µ anfällt, durch Filtration abgetrennt, mit Wasser (350 ml) gewaschen, zentrifugiert und an der Luft oder in einem entsprechend eingestellten Fliessbett getrocknet. Aus 250 g feuchtem Produkt erhält man 219,12 g (99,4 %) Trockenprodukt mit folgenden Eigenschaften : $(C_9H_6ON)_2Cu$ = 90,45 % ; $H_2O$ = 9,45 % ; Gesamtgehalt an Verunreinigungen (Oxinate anderer Metalle, Ammoniumsulfat, Oxin, Wasser) : ≤ 0,80 % ; Polymorphreinheit (β'), festgestellt durch Röntgenbeugung :

# 0 069 310

100 % ; Lage der Hauptlinien der Röntgenbeugung in Dezimalgraden : (2 θ) = 6,95 ; 15,75 ; 16,00.

Die Mutterlauge und die Waschwasser (3 Liter) werden auf einen pH von 7-8 (mit etwa 50 ml Ammoniak) eingestellt und 12 Stunden stehengelassen. Der anfallende Niederschlag, welcher hauptsächlich aus Eisenoxinat (etwa 0,450 g) besteht, kann nur schwer durch Filtration, aber in einfacher Weise durch Zentrifugation abgetrennt werden.

### Beispiel 2

Herstellung der polymorphen Form β' $(C_9H_6ON)_2Cu$

Die Trocknung des feuchten Kupferoxinats in β-Form erfolgt in zwei hintereinander folgenden Stufen :

(I) Feuchtes Oxinat β → Oxinat β + Feuchtigkeit ;

(II) Oxinat β → Oxinat β' + $2H_2O$.

Wie bereits ausgeführt, erfolgt die Trocknung (I) bereits bemerkenswert gut an der Luft. In einer Fliessbetttrockenanlage, bei welcher die Lufteintrittstemperatur auf 40 °C eingestellt ist, wird die Feuchtigkeit innerhalb von 10 Minuten vollständig entfernt, wobei das Ausmass der Transformation der zweiten Stufe so gering ist, dass es nicht wahrnehmbar ist. Um diese zweite Stufe in einer vernünftigen Zeitspanne durchführen zu können, muss die Trocknung bei einer Temperatur von 85 °C während 10 Minuten erfolgen.

Man kann auch das trockene Kupferoxinat in β-Form während einer Pulverisierung in Heissluft behandeln, wobei das Oxinat während einer Zeitspanne in der Grössenordnung einer Sekunde bei einer Temperatur von 135-145 °C gehalten wird.

Die Reinheit des erhaltenen Produktes ist im allgemeinen wie folgt : β-Form = nicht feststellbar ; β''-Form ≤ 1 %. Der erhaltene Feststoff ist grün gefärbt, stark elektrostatisch und weist im Elektronenmikroskop eine fadenförmige Mikrostruktur auf.

Lage der Hauptlinien der Röntgenbeugung in Dezimalgrad : (2 θ) = 7,15 ; 11,95 ; 13 ; 14,7.

### Beispiel 3

Zur Deutlichmachung des Einflusses von Stabilisierungsmitteln auf die Stabilität des Kupferoxinats in β'-Form wurden die folgenden Präparate geprüft :

Präparat A

Kupferoxinat in β'-Form, enthaltend 1 Gewichtsprozent Sulfoxin. (Sulfoxin ist die freie 8-Hydroxychinolin-5-sulfonsäure oder deren Natriumsalz).

Präparat B

Kupferoxinat in β'-Form, enthaltend 10 Gewichtsprozent Natriumlignosulfonat.

Präparat C

Kupferoxinat in β'-Form, enthaltend 5 Gewichtsprozent Tridecylalkohol-polyäthoxyäther (nicht stabilisiertes Präparat).

Diese Präparate wurden in Form von wässrigen Suspensionen mit einem Gehalt von 5 Gewichtsprozent an Kupferoxinat in β'-Form verwendet.

| | | | | | | | | % Umwandlung in β bei Raumtemperatur |
|---|---|---|---|---|---|---|---|---|
| Zeit | 0h15 | 1h | 2h30 | 7h | 20h | 90h | 190h | 6 Monate |
| 5%-ige wässrige Suspension von β'-Kupferoxinat | 4 | 22 | 52 | | 92 | 100 | | |
| Präparat A | 0 | | | | | | 0 | |
| Präparat B | 0 | | | | | | | 0 |
| Präparat C | | | 92 | | | | | |

5

| | % Umwandlung in β bei 60°C | | | |
|---|---|---|---|---|
| Zeit | 0h15 | 1h | 2h30 | 7h |
| 5%-ige wässrige Suspension von β'-Kupferoxinat | 0 | 100 | | |
| Präparat A | 0 | 0 | | 0 |

Hieraus ergibt sich das der Zusatz eines Stabilisierungsmittels die Stabilität des Kupferoxinats in β'-Form in wässriger Lösung stark erhöht, während die Zugabe eines üblichen Befeuchtungsmittels die Transformation in die β-Form beträchtlich beschleunigt.

## Beispiele 4-6

Die folgenden Versuche dienen zur Veranschaulichung der Wirkung von Kupferoxinat in β'-Form beim Schutz von Saatgut.

Diese Untersuchungen wurden in Uebereinstimmung mit den Protokollen der Commission des Essais Biologiques de la Société Française de Phytiatrie et de Phytopharmacie durchgeführt.

## Beispiel 4

Die verwendeten Formulierungen haben die folgenden Zusammensetzungen :

| Präparat D | Gewichtsprozent |
|---|---|
| Kupferoxinat β | 16,60 |
| Tridecylalkohol-polyäthoxyäther | 0,75 |
| Calciumcarbonat (Kreide) | 29,20 |
| Kaolin | 53,45 |

| Präparat E | Gewichtsprozent |
|---|---|
| Kupferoxinat β' | 15,00 |
| Tridecylalkohol-polyäthoxyäther | 0,75 |
| Calciumcarbonat (Kreide) | 29,20 |
| Kaolin | 50,05 |

Die Präparate wurden in einer Dosierung verwendet entsprechend 30 g/Zentner Kupferoxinat als $(C_9H_6NO)_2Cu$.

Die Behandlung des Saatgutes erfolgte sofort nach Herstellung der Brühen.

Vergleich der Wirksamkeit der polymorphen Formen β und β' gegen Fusariose, Septoriose und Getreidebrand

Die erhaltenen Resultate sind in der Tabelle I zusammengefasst.

(Siehe Tabelle I Seite 7 f.)

Tabelle I

Prüfung auf Wirkung gegen Fusariose, Septoriose und Getreidebrand

% gesunde Pflanzen

| Art der Prüfung | in Pflanzschalen | | im offenen Feld |
|---|---|---|---|
| Krankheit | Fusariose(1) | Septoriose(2) | Getreidebrand |
| Tag der Zählung | S + 53 | S + 83 | Ernte |
| Präparat | | | |
| Präparat B | 74 | 25 | 92,8 |
| Präparat E | 85 | 68,5 | 97 |
| Kontrolle | 48 | 6 | 49,3 |

(1) Roggen zu 32 % mit Fusarium nivale kontaminiert.
(2) Weizen Clement zu 81 % mit Septoria nodorum kontaminiert.
(3) Weizen Clement zu 3 ‰ künstlich mit Getreidebrand kontaminiert.

Die obigen Resultate zeigen eine starke Ueberlegenheit in der Wirkung der Form β' gegenüber der Form β.

Beispiel 5

Die verwendeten Formulierungen hatten die folgenden Zusammensetzung :

Präparat G
Kupferoxinat β mit einem Gehalt von 5,55 Gew./Vol. % in Wasser.

Präparat H
Kupferoxinat β' mit einem Gehalt von 5 % Gew./Vol. in Wasser.

Präparat I

| | |
|---|---:|
| Kupferoxinat β' | 5 g |
| Natriumlignosulfonat | 0,5 g |
| Wasser, ad | 100 ml. |

Die Präparate wurden in einer Dosierung entsprechend 30 g/Zentner Kupferoxinat als $(C_9H_6NO)_2Cu$ verwendet.

Falls nicht anders angegeben, so erfolgte die Behandlung des Saatgutes unmittelbar nach der Herstellung der Brühen.

Nachdem in Beispiel 4 die erhöhte Wirksamkeit der β'-Form gegenüber der β-Form dargelegt wurde, soll die folgende Untersuchung die stabilisierende Wirkung auf die β'-Form nach 24 Stunden in Gegenwart von Wasser zeigen.

Vergleich der Wirksamkeit der polymorphen Formen β und β' gegen Fusariose, Septoriose, Getreidebrand und Helminthosporiose

Stabilisierende Wirkung

Die erhaltenen Resultate sind in der Tabelle II zusammengestellt.

Tabelle II
Prüfung auf die Wirksamkeit gegen Fusariose, Septoriose, Getreidebrand und Helminthosporiose

Stabilisierende Wirkung                                    Prozent gesunder Pflanzen

| Art der Prüfung / Krankheit / Datum der Zählung | in Pflanzschalen | | auf dem offenen Feld | |
|---|---|---|---|---|
| | Fusariose [1] | Septoriose [2] | Getreidebrand [3] | Helminthosporiose [4] |
| | S + 23 | S + 55 | Ernte | Aehrenbildung |
| Präparat G | 69,3 | 14,7 | 94,9 | 80,4 |
| Präparat H | 82 | 81,3 | 99,6 | 97,8 |
| Präparat H nach 24 Stunden | 75,3 | 40 | – | – |
| Präparat I | 80 | 78,3 | 98,9 | 98,8 |
| Präparat I nach 24 Stunden | 82,7 | 76,7 | 99,6 | 99,1 |
| nicht behandelte Kontrolle | 63,3 | 5,3 | 80,6 | 63,6 |

[1] Roggen zu 26 % mit Fusarium nivale kontaminiert.
[2] Weizen Talent zu 75 % mit Septoria nodorum kontaminiert.
[3] Weizen Clement zu 3 ‰ künstlich mit Getreidebrand kontaminiert.
[4] Wintergerste Astrix zu 56 % mit Helminthosporium gramineum kontaminiert.

Aus der Tabelle II ergibt sich deutlich, dass die nicht stabilisierten wässrigen Präparate enthaltend β′ Kupferoxinat während 24 Stunden einen Grossteil ihrer Aktivität verlieren, während die stabilisierten Präparate ihre Stabilität praktisch unverändert beibehalten.

Beispiel 6

Die verwendeten Präparate D und E hatten die in Beispiel 4 angegebenen Zusammensetzung, während die Präparate J-L die folgenden Zusammensetzungen hatten:

| Präparat J | Gewichtsprozent |
|---|---|
| Kupferoxinat β′ | 15,00 |
| Natriumpolynaphthylmethansulfonat | 1,00 |
| Sulfoxin | 0,2 |
| Calciumcarbonat (Kreide) | 29,2 |
| Kaolin | 54,6 |

| Präparat K | g/l |
|---|---|
| Kupferoxinat β | 133,3 |
| Lindan | 200 |
| Anthrachinon | 200 |
| Tridecylalkohol-Polyäthoxyäther | 10 |
| Nonylphenylpolyäthoxyäther | 15 |
| Rouge 53-1 | 35 |
| Aethylenglykol | 50 |
| Wasser, ad | 1 Liter |

| Präparat L | g/l |
|---|---|
| Kupferoxinat β′ | 120 |
| Lindan | 200 |
| Anthrachinon | 200 |
| Rouge 53-1 | 35 |
| Sulfoxin | 10 |
| Natriumpolynaphthylmethansulfonat | 20 |
| Polyvinylalkohol | 50 |
| Aethylenglykol | 50 |
| Wasser, ad | 1 Liter |

Die Präparate wurden in einer Dosierung entsprechend 30 g/Zentner Kupferoxinat, ausgedrückt als $(C_9H_6NO)_2Cu$, verwendet.

Wenn nicht anders angegeben, erfolgte die Behandlung des Saatgutes unmittelbar nach der Herstellung der Brühe.

Untersuchung der Wirksamkeit von β′ Kupferoxinat in Gegenwart bzw. Abwesenheit von Stabilisierungsmitteln, welche gegebenenfalls noch andere Wirkstoffe enthalten

Die Ergebnisse dieser Untersuchungen sind in Tabelle III zusammengestellt.

Tabelle III
Prüfung auf Wirkung gegen Septoriose Stabilisierungseffekt

Prozent gesunder Pflanzen

| Art der Untersuchung | Pflanzschalen |
|---|---|
| Krankheit / Präparat | Septoriose (1) |
| **Formulierungen mit 3 Wirkstoffen** | |
| Präparat D | 69,8 |
| Präparat E nach 24 Stunden | 65,3 |
| Präparat J nach 24 Stunden | 91,6 |
| nicht behandelte Kontrolle | 9,8 |
| **Einfache Formulierung** | |
| Präparat K | 40,4 |
| Präparat L nach 24 Stunden | 81,2 |
| nicht behandelte Kontrolle | 9,5 |

(1) Weizen Talent zu 90 % mit Septoria nodorum kontaminiert.

Die in der Tabelle III enthaltenen Resultate bestätigen die stabilisierende Wirkung auf die polymorphe Form β'. Diese Wirkung wird durch die Anwesenheit anderer Wirkstoffe nicht beeinträchtigt.

Die obigen Untersuchungen zeigen ebenfalls eine wesentlich verbesserte Wirksamkeit der Form β' gegenüber der Form β.

Die obigen Untersuchungen zeigen in ihrer Gesamtheit das beträchtliche Interesse an der Form β' im Hinblick auf ihre Wirksamkeit gegen Getreidesaatgutkrankheiten.

**Patentansprüche**

1. Verfahren zur Herstellung von Kupferoxinat in β'-Form, dadurch gekennzeichnet, dass man :
   a) ein Säureadditionssalz von 8-Hydroxychinolin mit einem Cu (II)-Salz in Wasser umsetzt,
   b) eine Base zusetzt bis der pH zwischen 2 und 9, vorzugsweise zwischen 2 und 3,5 liegt ;
   c) den gebildeten Niederschlag abtrennt ; und
   d) den so abgetrennten Niederschlag bei einer Temperatur zwischen 40 und 220 °C dehydratisiert.

2. Verfahren nach Anspruch 1, dadurch gekennzeichnet, dass man in der Stufe b) den pH-Wert auf 2,5 bis 2,8 einstellt.

3. Verfahren nach Anspruch 1 oder 2, dadurch gekennzeichnet, dass man die Stufe d) bei einer Temperatur zwischen 60 und 160 °C während einer Zeitdauer zwischen 120 Minuten und 0,5 Sekunden durchführt.

4. Verfahren nach Anspruch 3, dadurch gekennzeichnet, dass man die Stufe d) bei einer Temperatur zwischen 80 und 140 °C während einer Zeitdauer zwischen 20 Minuten und 0,5 Sekunden durchführt.

5. Verfahren nach einem der Ansprüche 1-4, dadurch gekennzeichnet, dass man in der Stufe a) Kupfer (II)-Sulfat mit Oxinsulfat umsetzt.

6. Verfahren nach einem der Ansprüche 1-5, dadurch gekennzeichnet, dass man in der Stufe b) als Base Ammoniak verwendet.

7. Verwendung von Kupferoxinat in β'-Form als Antifungusmittel.

8. Verwendung gemäss Anspruch 7 für den Schutz von Saatgut.

9. Stabilisiertes β'-Kupferoxinatpräparat, dadurch gekennzeichnet, dass es als Hydratationsinhibitor mindestens eine die β'-Form von Kupferoxinat stabilisierende Substanz enthält.

10. Präparat nach Anspruch 9, dadurch gekennzeichnet, dass es als Hydratationsinhibitor eine oder mehrere der folgenden Substanzen enthält : niedermolekulare Polykondensate vom Kresolphenoplasttyp und vom Formaldehydphenoplasttyp, Polykondensate von Alkylnaphthalinsulfonsäuren mit Formaldehyd und deren Salze, Arylsulfonsäure und deren Salze, Polyacrylsäure, sowie deren Homologe und deren Salze, Lignosulfonsäure und Lignosulfonate.

11. Präparat nach Anspruch 9 oder 10, dadurch gekennzeichnet, dass es als Hydratationsinhibitor eine oder mehrere der folgenden Substanzen enthält : Sulfoxin (8-Hydroxy-chinolin-5-sulfonsäure und deren Salze, sowie Halogensulfoxine, insbesondere 7-Jod-8-hydroxychinolin-7-sulfonsäure und deren Salze.

12. Präparat nach Anspruch 9, dadurch gekennzeichnet, dass es als Hydratationsinhibitor eine oder mehrere der folgenden Substanzen enthält : Polyvinylalkohol, Alkylcellulosen, Hydroxyalkylcellulosen, Carboxyalkylcellulosen, Polyvinylpyrrolidon, Polyacrylamide, Saccharide wie Stärken, Hemicellulosen, Gummen, Polypeptide und insbesondere Casein und Caseinate.

## Claims

1. A process for the manufacture of copper oxinate in the $\beta'$-form, characterized by :
   a) reacting an acid addition salt of 8-hydroxyquinoline with a Cu (II) salt in water,
   b) adding a base until the pH lies between 2 and 9, preferably between 2 and 3.5 ;
   c) separating the precipitate formed ; and
   d) dehydrating the thus-separated precipitate at a temperature between 40 and 220 °C.

2. A process according to claim 1, characterized in that the pH value is adjusted to 2.5 to 2.8 in step b).

3. A process according to claim 1 or 2, characterized in that step d) is carried out at a temperature between 60 and 160 °C for a period between 120 minutes and 0.5 seconds.

4. A process according to claim 3, characterized in that step d) is carried out at a temperature between 80 and 140 °C for a period between 20 minutes and 0.5 seconds.

5. A process according to any one of claims 1-4, characterized in that copper (II) sulphate is reacted with oxine sulphate in step a).

6. A process according to any one of claims 1-5, characterized in that ammonia is used as the base in step b).

7. The use of copper oxinate in the $\beta'$-form as an antifungal agent.

8. The use in accordance with claim 7 for the protection of seeds.

9. A stabilized $\beta'$-copper oxinate preparation, characterized in that it contains as a hydration inhibitor at least one substance which stabilizes the $\beta'$-form of copper oxinate.

10. A preparation according to claim 9, characterized in that it contains one or more of the following substances as the hydration inhibitor : low-molecular polycondensates of the cresol-phenoplast type and of the formaldehyde-phenoplast type, polycondensates of alkylnaphthalene sulphonic acids with formaldehyde and their salts, arylsulphonic acids and their salts, polyacrylic acids, as well as their homologues and their salts, lignosulphonic acids and lignosulphonates.

11. A preparation according to claim 9 or 10, characterized in that it contains one or more of the following substances as the hydration inhibitor : sulphoxin (8-hydroxyquinoline-5-sulphonic acid and its salts, as well as halosulphoxins, especially 7-iodo-8-hydroxyquinoline-7-sulphonic acid and its salts.

12. A preparation according to claim 9, characterized in that it contains one or more of the following substances as the hydration inhibitor : polyvinyl alcohol, alkylcelluloses, hydroxyalkylcelluloses, carboxyalkylcelluloses, polyvinylpyrrolidone, polyacrylamides, saccharides such as starches, hemicelluloses, gums, polypeptides and especially casein and caseinates.

## Revendications

1. Procédé de préparation de l'oxinate de cuivre sous la forme $\beta'$, caractérisé en ce que :
   a) on fait réagir un sel de la 8-hydroxyquinoléine formé par addition avec un acide avec un sel de Cu-II dans l'eau ;
   b) on ajoute une base en quantité suffisante pour porter le pH entre 2 et 9, de préférence entre 2 et 3,5 ;
   c) on sépare le précipité formé ; et
   d) on déshydrate ce précipité à une température de 40 à 220 °C.

2. Procédé selon la revendication 1, caractérisé en ce que, au stade opératoire b), on règle le pH à un niveau de 2,5 à 2,8.

3. Procédé selon la revendication 1 ou 2, caractérisé en ce que l'opération du stade d) est réalisée à une température de 60 à 160 °C en une durée de 120 minutes à 0,5 seconde.

4. Procédé selon la revendication 3, caractérisé en ce que l'opération du stade d) est réalisée à une température de 80 à 140 °C en une durée de 20 minutes à 0,5 seconde.

5. Procédé selon l'une des revendications 1 à 4, caractérisé en ce que, au stade opératoire a), on fait réagir le sulfate de cuivre-II avec le sulfate d'oxine.

6. Procédé selon l'une des revendications 1 à 5, caractérisé en ce que, au stade b), la base utilisée est l'ammoniaque.

7. Utilisation de l'oxinate de cuivre sous la forme $\beta'$ en tant qu'agent antifongique.

**0 069 310**

8. Utilisation selon la revendication 7 pour la protection des semences.

9. Composition stabilisée d'oxinate de cuivre β', caractérisée en ce qu'elle contient en tant qu'inhibiteur d'hydratation au moins une substance stabilisant la forme β' de l'oxinate de cuivre.

10. Composition selon la revendication 9, caractérisée en ce qu'elle contient en tant qu'inhibiteur d'hydratation une ou plusieurs des substances suivantes ; des polycondensats à bas poids moléculaire du type phénoplaste de crésol et du type phénoplaste de formaldéhyde, des polycondensats d'acides alkylnaphtalène-sulfoniques avec le formaldéhyde et leurs sels, un acide arylsulfonique et ses sels, l'acide polyacrylique, ses homologues et ses sels, l'acide lignosulfonique et des lignosulfonates.

11. Composition selon la revendication 9 ou 10, caractérisée en ce qu'elle contient en tant qu'inhibiteur d'hydratation une ou plusieurs des substances suivantes ; la sulfoxine (acide 8-hydroxyquinoléine-5-sulfonique) et ses sels, et des halogénosulfoxines, en particulier l'acide 7-iodo-8-hydroxyquinoléine-7-sulfonique et ses sels.

12. Composition selon la revendication 9, caractérisé en ce qu'elle contient en tant qu'inhibiteurs d'hydratation une ou plusieurs des substances suivantes : alcool polyvinylique, alkylcellulose, hydroxyalkylcelluloses, carboxyalkylcelluloses, polyvinylpyrrolidone, polyacrylamides, des saccharides tels que les amidons, des hémicelluloses, des gommes, des polypeptides et en particulier la caséine et des caséinates.

13